# EUROPEAN PATENT APPLICATION

(11) **EP 4 147 640 A1**
(43) Date of publication of application: **15.03.2023**
(21) Application number: 22194078.6
(22) Date of filing: 06.09.2022
(51) Int. Cl.: A61B 5/20, G01G 1/00, A61B 10/00, A61B 5/145, A61B 5/1468, G01G 17/04, G01N 33/50

(54) **URINE ANALYSIS DEVICE**

(30) Priority: 14.09.2021 IT 202100023663
(71) Applicant: Kures S.r.l., 20122 Milano (IT)
(72) Inventor: Orsenigo, Renzo, MILANO (IT); Manzoni, Giulia, MILANO (IT); Manzoni, Angelo, MILANO (IT); Poretti, Ezio, MILANO (IT)
(74) Representative: Lunati & Mazzoni S.r.L.

(57) **Abstract**

It is provided a urinalysis device (1) capable of analysing a urine sample to determine the value of physico-chemical characteristics of the urine sample and comprising a first sampler module (2) configured to be placed in fluid passage connection with a user and comprising a first drain drainage duct (20) comprising a containment part (20a) defining a predetermined volume and configured to hold a first portion of urine, and a release part (20b) configured to convey a second portion of urine out of the first sampler module (2); a diuresis bag (3) in fluid passage connection with at least the release part (20b) and configured to collect the second portion of urine; an analysis apparatus (4) placed in fluid passage connection with the containment part (20a) and configured to analyse at least part of the first urine portion; control means (5) operatively connected to the analysis apparatus (4) and configured at least to process data collected by the analysis apparatus (4); and a second sampler module (7) operatively connected to the analysis apparatus (4) and including an shut-off valve (70) in fluid passage connection with the analysis apparatus (4) and configured to accommodate a syringe engagement including sampled urine so that the analysis apparatus (4) can analyse the sampled urine.

## Description

The present invention relates to a urine analysis device of the type specified in the preamble to the first claim.

The device, according to the invention, can be placed in the vicinity of a bed, for example at least partially below the bed, and can be connected via a catheter directly to the patient. In detail, the device is capable of performing an electrochemical urine analysis.

As is well known, the urine examination is currently one of the main methods of analysis to demonstrate or exclude many pathologies or problems such as, for example, structural lesions, the presence of infectious agents or even an alteration of one or more organs. In particular, the urine test is the main form of kidney analysis and, therefore, is an important test for recognising possible kidney dysfunction and, thus, the state of the kidneys themselves.

One of the main types of analysis is electrochemical analysis in which the potential difference between a pair of electrodes is calculated and, based on this, the pH, sodium, potassium, ammonium and chloride content within the urine is determined. This analysis is currently carried out as described below.

A catheter is attached to the patient, which allows urine to be pumped into a special container, such as a bag, from which medical personnel draw the correct amount of urine. This can be done either by means of a syringe, or other similar instrument, appropriately inserted into the bag, or by means of an appropriate dropper to measure the correct amount of urine.

The quantity taken is then diluted, so as to reduce the interference of interfering substances that can lead to incorrect sample evaluations and, therefore, to an incorrect analysis.

Once diluted, the urine sample is inserted into the analysis device which, through a certain procedure, performs the analysis.

In detail, reagents and/or chemical solutions are added to the urine sample to counteract the negative influence of the presence of disturbing substances that may lead to incorrect analysis. These added elements vary depending on the analysis to be performed and, for example, may include the urease enzyme used for urea analysis and the cation exchange column required to remove or decrease the high ammonium ion content.

Once the sample is ready to be analysed, it is placed, for example, between two electrodes and, by measuring the potential difference between these electrodes, the content of the substance sought can be determined by performing the analysis. The well-known technique mentioned above has some major drawbacks.

In fact, the procedure used is particularly lengthy and requires the continuous presence of medical personnel. In particular, the collection of the amount of urine to be analysed, carried out by the operator, results in a possible contamination of the sample in addition to an obvious increase in examination time.

Another problem is the analysis itself, which is particularly complicated and difficult to perform.

In particular, the content of urine, being extremely variable both from patient to patient and between different samples taken from the same patient, requires different dilution with reagents from time to time.

Another critical aspect is that the analysis is also a function of temperature, which has a decisive influence on the final result.

These aspects therefore result in a difficult adjustment of all parameters and, consequently, lead to a sub-optimal analysis of the urine sample.

In this situation, the technical task at the basis of the present invention is to devise a urine analysis device capable of substantially obviating at least some of the aforementioned drawbacks.

Within the scope of this technical task, it is an important aim of the invention to devise a device that allows simple, fast and frequent analyses and involves substantial monitoring of the patient's condition.

In fact, the fundamental organs for survival are the heart, lungs and kidneys, and while there are various machines to monitor the heart and lungs, by analysing the respiratory and cardiovascular systems, the kidneys cannot be monitored with known machines.

A further aim of the invention is to realise a device that does not require the presence of medical and paramedical personnel and, therefore, a device that allows analyses to be carried out essentially automatically.

The specified technical task and purposes are achieved by a urine analysis device as claimed in the annexed claim 1.

Preferred technical solutions are highlighted in the dependent claims.

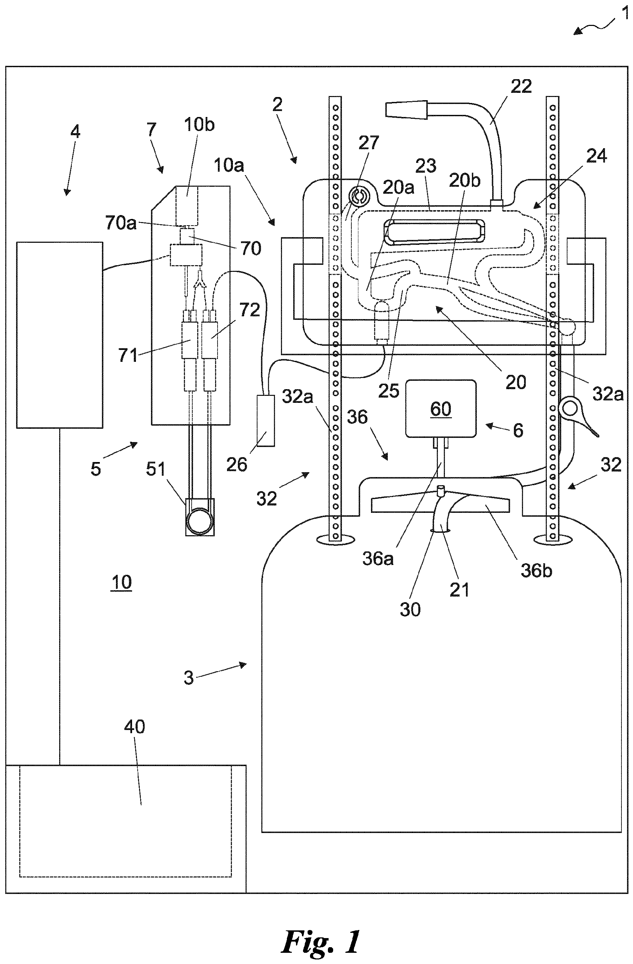

The features and advantages of the invention are clarified below by a detailed description of preferred embodiments of the invention, with reference to the accompanying drawings, in which
the **Fig. 1** shows a schematic frontal view of a urine analysis device according to the invention;

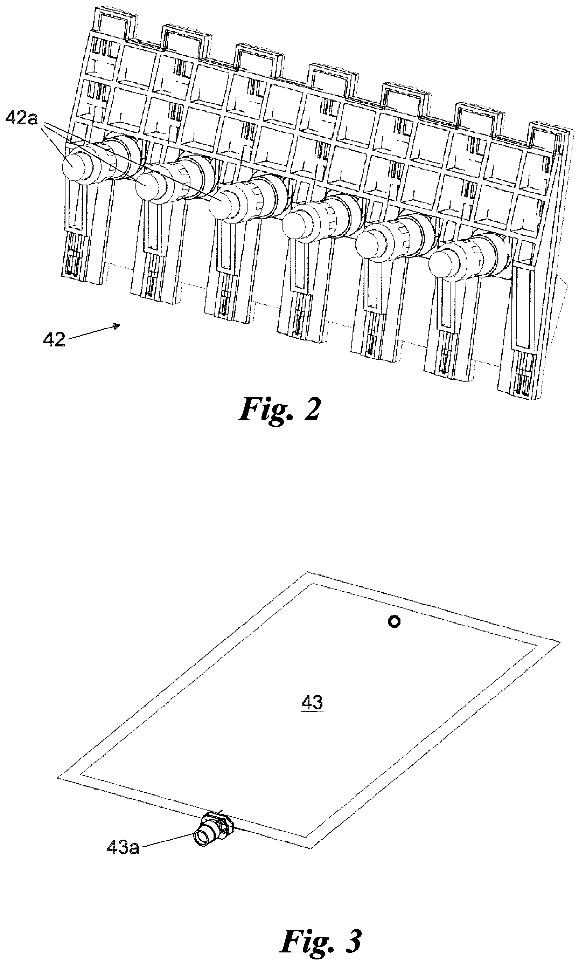

the **Fig. 2** illustrates the detail of the rigid rack forming the interface surface of a cartridge of a urine analysis device according to the invention;
the **Fig. 3** is a detail of the bag of a cartridge of a urine analysis device according to the invention;
the **Fig. 4a** represents a perspective view of a cartridge of a urine analysis device according to the invention in which the opening extends over the entire interface surface;
the **Fig. 4b** shows a perspective view of a cartridge of a urine analysis device according to the invention in which the opening extends over part of the interface surface in particular at the plugs and includes a cover;

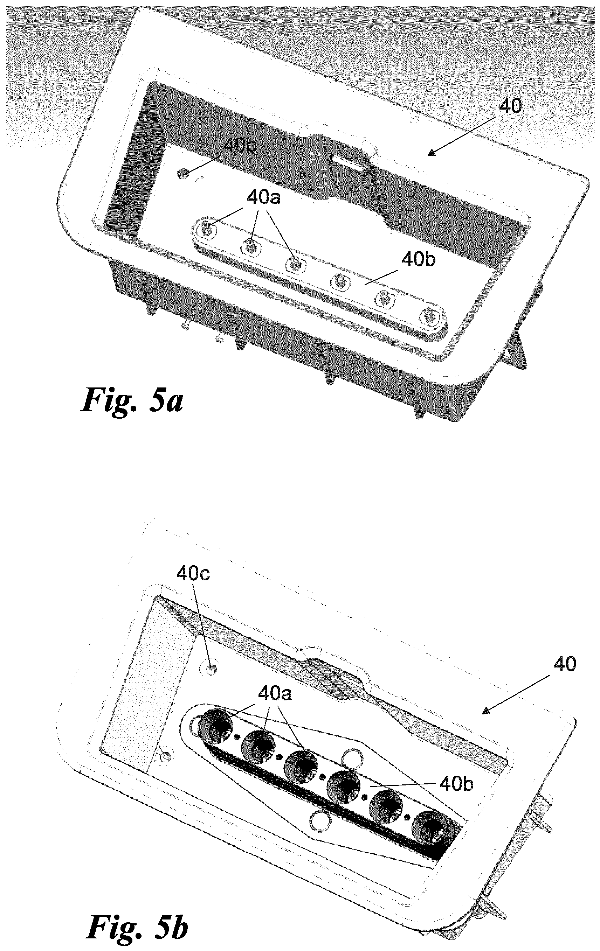

the **Fig. 5a** illustrates the detail of the interface seat of a urine analysis device cartridge according to the invention in which the drain hole at the bottom is visible and in which a guide with pins is present;
the **Fig. 5b** is a detail of the interface seat of a cartridge of a urine analysis device according to the invention in which several drain holes are visible at the bottom and in which a guide with inlets is present;
the **Fig. 6** depicts a front view of the analysis station of a urine analysis device according to the invention; and

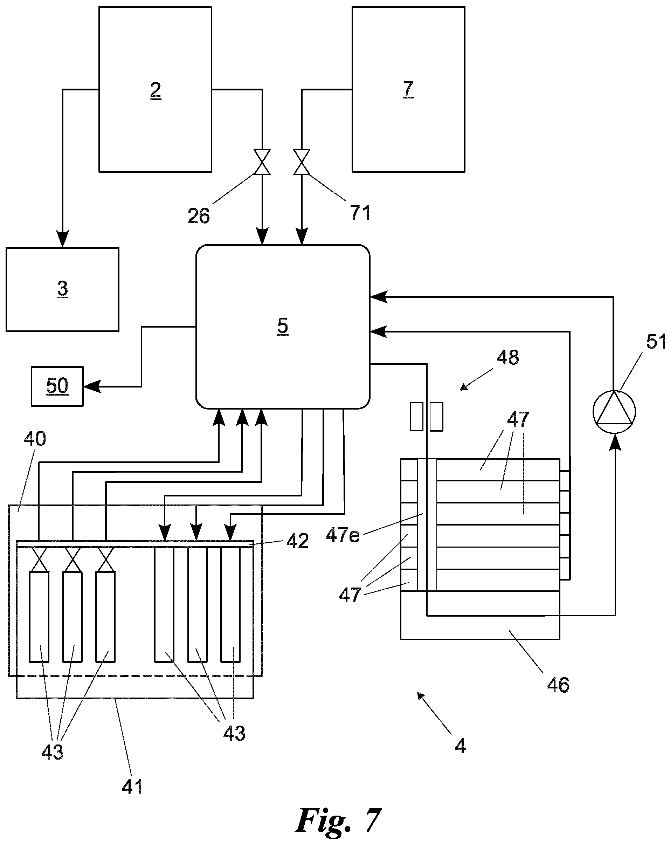

the **Fig. 7** shows a simplified functional diagram of a urine analysis device according to the invention.

In the present document, the measurements, values, shapes and geometric references (such as perpendicularity and parallelism), when associated with words like "about" or other similar terms such as "approximately" or "substantially", are to be considered as except for measurement errors or inaccuracies due to production and/or manufacturing errors, and, above all, except for a slight divergence from the value, measurements, shape, or geometric reference with which it is associated. For instance, these terms, if associated with a value, preferably indicate a divergence of not more than 10% of the value.

Moreover, when used, terms such as "first", "second", "higher", "lower", "main" and "secondary" do not necessarily identify an order, a priority of relationship or a relative position, but can simply be used to clearly distinguish between their different components.

Unless otherwise specified, as results in the following discussions, terms such as "treatment", "computing", "determination", "calculation", or similar, refer to the action and/or processes of a computer or similar electronic calculation device that manipulates and/or transforms data represented as physical, such as electronic quantities of registers of a computer system and/or memories in, other data similarly represented as physical quantities within computer systems, registers or other storage, transmission or information displaying devices.

The measurements and data reported in this text are to be considered, unless otherwise indicated, as performed in the International Standard Atmosphere ICAO (ISO 2533:1975).

With reference to the Figures, the urine analysis device according to the invention is collectively referred to as **1.**

In particular, it is capable of being used for the analysis of a urine sample, preferably as it is, i.e. without the addition of reagents or solutions, by determining the value of its physico-chemical characteristics. More specifically, the device 1 determines the pH and the sodium, potassium, ammonium and chloride contents of a urine sample and, at the same time, determines its instantaneous urine flow.

The device 1 comprises at least a first sampler module **2.**

The first sampler module 2 is part of the inlet system for placing the fluid to be analysed inside the device 1. In particular, the first sampler module 2 is configured to be placed in fluid passage connection with a user.

Thus, the first sampler module 2 allows the urine to reach device 1 as it exits, and to dose the fluid entering the device 1.

In this regard, in more detail, the first sampler module 2 preferably comprises a first drainage duct **20.** The first drainage duct 20 is the channel through which the urine passes in order to be handled by the device 1 before being disposed of.

Preferably, the first drainage duct 20 is a syphonic conduit including a coil that allows the flow of fluid, i.e. urine, to be managed automatically.

Therefore, the first drainage duct 20 comprises, preferably, a containment part **20a** and a releasing part **20b.**

The containment part 20a is essentially a portion of the first drainage duct 20 arranged upstream of the release part 20b.

The containment part 20a is, in fact, configured to retain a first portion of urine. For these purposes, the containment part 20a may substantially comprise a section of first U-shaped drainage duct 20 preceding the release part 20b. The latter is, instead, configured to convey a second portion of urine out of the first sampler module 2. Basically, when urine flows into the first drainage duct 20, it passes through the containment part 20a and, having reached a sufficient level of filling, begins to flow through the releasing part 20b, at least in part, so that it is partly expelled by the first sampler module 2.

In addition, the containment part 20a defines a predetermined volume. Thus, it is possible to know the volume of the first portion of urine that stops in the first sampler module 2.

The first sampler module 2 may include additional features.

In particular, the first sampler module 2 may include a cannula outlet **21.**

If present, the cannula outlet 21 is in fluid passage connection with the release part 20b. Thus, the cannula outlet 21 is suitable for conveying the urine out of the first sampler module 2 in a controlled manner.

The first sampler module 2 can also include a cannula inlet **22.**

If present, the cannula inlet 22 is configured to be connected to the user. Thus, the cannula inlet 22 is the part of the first sampler module 2 that is to be directly connected to the user.

In addition, the first sampler module 2 can also include a first tank **23.**

The first tank 23 is essentially a collection tank for collecting urine from the patient. Therefore, preferably, the first tank23 is in fluid passage connection with the cannula inlet 22 and the containment part 20a. In other words, the first tank 23 is arranged between the cannula inlet 22 and the containment part 20a and is suitable for receiving the urine prior to its entry into the containment part of the first drainage duct 20.

In addition, the first tank 23 may comprise an inclined base plane configured, when the first sampler module 2 is in use, to push the urine to flow, by gravity, towards the first drainage duct 20.

The first sampler module 2 may also comprise a second drainage duct **24.** The second drainage duct 24 may be a channel developed in parallel with the first drainage duct 20. In further detail, the second drainage duct 24 is in fluid passage connection with the first tank 23 and the cannula outlet 21. The second drainage duct 24 is configured to convey urine from the first tank 23 to the cannula outlet 21. Preferably, the second drainage duct 24 draws urine from the first tank 23 when the urine in the first tank 23 exceeds a predetermined threshold level.

Therefore, in essence, the second drainage duct 24 is a safety or empty-full channel configured to prevent the first tank 23 from filling abnormally, altering the proper functioning of the first sampler module 2.

In addition, the tank 23 may comprise a filtration mesh at the upper, relative to the ground, portion of the tank 23, i.e., close to the cannula inlet 22. The filtration mesh, if present, comprises meshes of such a size as to allow the separation of foreign bodies from the urine such as, for example, clots, organic sedimentations or the like. The device 1 therefore also includes a diuresis bag **3.**

The bag 3 is basically a tank, e.g. delimited by deformable or even rigid or partly deformable and partly rigid walls, within which a fluid can be accommodated.

Therefore, the bag 3 is impermeable to liquids. Furthermore, the bag 3 is preferably in fluid passage connection with the release part 20b. In this way, therefore, the bag 3 is configured to collect the second urine portion, i.e. the urine output portion from the first sampler module 2.

The connection between bag 3 and release part 20b may be direct or indirect. Preferably, the connection between bag 3 and release part 20b is made by the cannula outlet 21.

In addition, bag 3 is advantageously decoupled from the first sampler module 2. Preferably, bag 3 includes a nozzle **30.**

The nozzle 30 is configured to accommodate at least part of the cannula outlet 21. Thus, the nozzle 30 is also substantially configured to make the fluid passage connection between the first sampler module 2 and the bag 3.

The outlet 30 may be a simple hole made in a wall, e.g. top, of the bag 3. In fact, preferably, the first module 2 is intended, in use, to be positioned above the bag 3, relative to the ground, so that the urine can flow by gravity into the bag 3. Alternatively, the nozzle 30 may include particular means of constraint, for example a quick connector capable of locking one end of the exit cannula 21 solidly to the wall of the bag 3, or a pre-drilled membrane configured to deform so as to allow part of the exit cannula 21 to enter the bag 3.

The device 1 also includes an analysis apparatus **4.**

The analysis apparatus 4, itself known, is configured to analyse at least part of the first urine portion. Therefore, the analysis apparatus 4 is placed in fluid passage connection with the containment part 20a.

Naturally, the device 1 also includes control means 5.

The control means 5 are operatively connected to at least the analysis apparatus 4. Furthermore, they are configured at least to process data collected by the analysis apparatus 4. Obviously, the control means 5 may also be operatively connected to other components of the device 1 as further specified below.

Preferably, in detail, the analysis apparatus 4 and the control means 5 may be similar to the analysis station and the command and control unit respectively as described in patent application EP-A-2510877 in paras. [0037-0052] incorporated herein by reference.

Essentially, the analysis apparatus 4 is suitable for carrying out an electrochemical analysis of urine and has a plurality of electrodes each of which is suitable for determining at least one parameter, for example a potential difference, for each of the physico-chemical characteristics. The analysis apparatus 4 may comprise an analysis station including at least five electrodes with the possibility of further expansion and, more specifically, a reference electrode **46** and at least one ion-selective electrode **47,** or other similar element capable of interacting with the substance to be analysed.

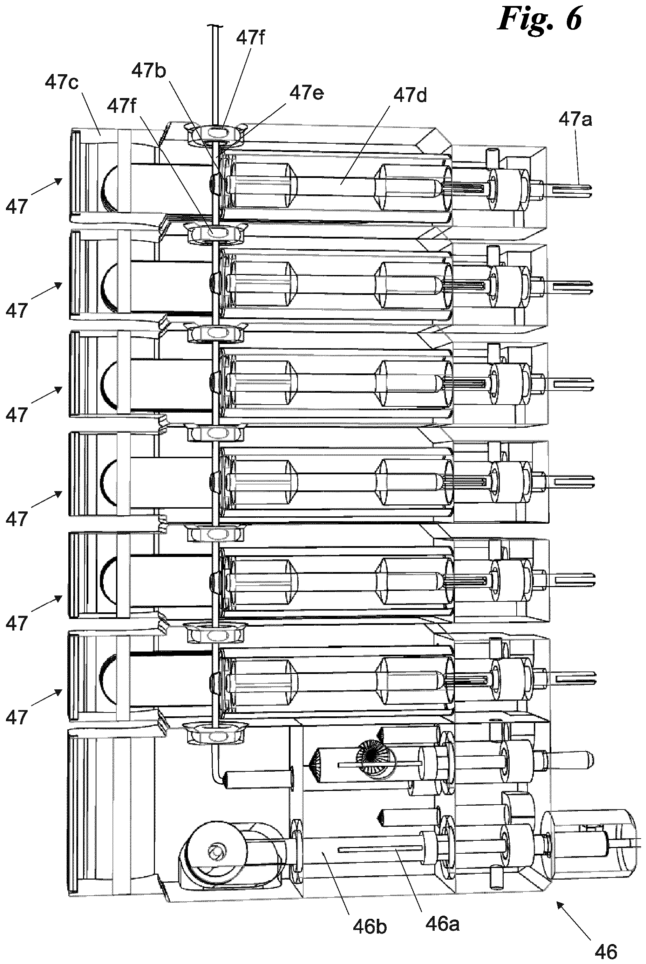

In detail, at least one ion-selective electrode 47 is provided for each of the following characteristics to be analysed: pH, sodium, potassium, ammonium and chloride contents. For example, Fig. 6 shows an analysis station equipped with six ion-selective electrodes 47, i.e. one for pH analysis, four for detecting the contents of sodium, potassium, ammonium and chloride, and a sixth which is not used for this particular analysis, but which can later be used to detect a further physico-chemical characteristic of a urine sample.

The reference electrode 46 has a conductive element **46a,** e.g. silver, housed in a chamber **46b** containing a fluid to maintain the electrode 46 at constant potential.

Each ion-selective electrode 47 is in contact with the medium and reacts with a specific ion. More in detail, the ion-selective electrode 47 has an electrical connector **47a,** for example a pin or even an electrical circuit, capable of enabling the electrical connection of the ion-selective electrode 47 with the standard electrode 46 in the presence of the fluid to be analysed; an ion-sensitive membrane **47b** or other similar element apt to react with a given ion, a central body **47c** capable of containing the almost all elements constituting the ion-selective electrode 47 and defining two distinct chambers a connection chamber **47d** interposed between membrane 47b and connector 47a and capable of containing a fluid characterising the electrode offset value, for example a saturated solution of KCI, and a containment chamber **47e** adjacent to the ion-selective electrode 47 and apt to contain urine.

In particular, the containment chamber 47e preferably comprises a through-hole so that, once the ion-selective electrodes 47 are in place, the containment chambers 47e form a single conduit in such a way as to allow a free passage of fluid both between said chambers and out of/into the analysis apparatus 4. In order to avoid fluid leakage, sealing elements, such as o-rings **47f,** may be interposed between said electrodes.

Each of the ion-selective electrodes 47 is capable of reacting with only one of the substances to be analysed. In detail, in the case of device 1 for determining pH and sodium, potassium, ammonium and chloride contents, the ion-selective electrodes 47 are sensitive to at least one of the following ions H+, Na+, K+, NH4+ and CI-. This selection is carried out by means of the ion-sensitive 47b membranes specially made and optimised for the element with which they are to interact.

For example, membranes for H+ and Na+ ions are glassy matrix, while membranes for K+, NH4+ and CI- ions are high molecular weight polymeric matrix.

The device 1 may also have, at the analysis apparatus 4 a thermostat **48** suitable for regulating the temperature of the fluid to be analysed and of the calibrator when the fluid is in the analysis apparatus 4. Said thermostat 48 comprises any element, for example an electrical resistance, suitable for maintaining the temperature of the urine substantially constant. Preferably, such temperature is substantially between 30°C and 45°C and, more preferably still, it is substantially 37°C.

Finally, each of the electrodes 46 and 47 is electrically connected to the control means 5, suitable for analysing the data collected by each ion-selective electrode 47, for comparing said collected data with that of the standard electrode 46, and for controlling, if present, a washing or calibration block suitable for allowing the preparation of the analysis apparatus 4 prior to the execution of an analysis.

The control means 5, in addition to analysing the data from the analysis apparatus 4 are preferably also connected to the first sampler module 2 and, more specifically, to first sensor means **25.** Indeed, one between the cannula inlet 22 and the first drainage duct 20 could comprise, in at least one embodiment, first sensor means 25. The latter are preferably optical means operatively connected to the control means 5 and configured to detect the passage of urine through the cannula inlet 22 or the first drainage duct 20.

Naturally, the first sampler module 2 may also further comprise a first pilot valve **26.** If present, the latter may also be operatively connected to the control means 5. Thus, the control means 5 may be configured to actuate the first pilot valve 26 when urine passes through the cannula inlet 22 or the first drainage duct 20.

The first pilot valve 26 may be substantially arranged between the analysis apparatus 4 and the first drainage duct 20, in particular with the containment part 20a, and configured to convey the first portion of urine from the containment part 20a to the analysis apparatus 4.

The first sampler module 2 preferably comprises a ventilation duct **27.** The ventilation duct 27 is preferably in fluid passage connection with two separate points of the first drainage duct 20 and with the external environment. More in detail, the ventilation duct 27 is placed in fluid passage connection with the ends of the containment part 20a and with the external environment. Thus, the ventilation duct 27 allows the urine collected in the containment part 20a to be subjected to the same pressures at both ends so that it can remain stably inside the syphonic portion, i.e. the containment part 20a.

The washing or calibration block can be made of removable components.

In a preferred, but not exclusive, form of realisation, the analysis apparatus 4, in addition to the analysis station, may comprise an interface seat **40.**

The interface seat 40 is preferably operatively connected to the analysis station including the electrodes. Furthermore, the interface seat 40 is essentially a tank, i.e. an open container within which removable components can be housed. Advantageously, the interface seat 40 comprising a plurality of inlets **40a.** Such inlets 40a may include orifices or plugs or any element permitting the insertion or overlapping of other couplable elements.

Preferably, the analysis apparatus also includes a **41** cartridge.

The cartridge 41 is an available element within the interface seat 40. In detail, cartridge 41 is preferably counter-shaped at least in part like interface seat 40.

In addition, cartridge 41 is suitable for interaction with inlets 40a.

Thus, advantageously, the cartridge 41 comprises an interface surface **42** and a plurality of bags **43.**

The interface surface 42 is the part of the cartridge 41 intended to interact directly with the inlets 40a. Therefore, preferably, the interface surface 42 comprises a plurality of plugs **42a.**

The plugs 42a are, therefore, configured to be coupled to inlets 40a. In detail, each 42a plug is coupled to a respective 40a inlet.

Naturally, in an alternative form of realisation, interface seat 40 could comprise plugs 42a and interface surface 42 could comprise inlets 40a.

In any case, cartridge 41 has a polarised mode of insertion into interface slot 40 as inlets 40a preferably each correspond to a specific plug 42a.

In addition, the plugs 42a or inlets 40a are configured to be coupled to inlets 40a or plugs 42a, respectively; furthermore, plugs 42a or inlets 40a of interface surface 42 are advantageously each in fluid passage connection with a respective bag 43. Each bag 43 is preferably impermeable to liquids. In addition, each bag includes a calibration or cleaning or control solution.

For example, the control solution is used to monitor the performance quality of the instrument or electrodes over time and follows the same procedure as an analysis.

Thus, the cartridge 41 advantageously contains all calibration and cleaning and control substances separate within itself and replaceable by coupling the respective bag 43 or removing it to the respective plug 42a or from the respective inlet 40a of interface surface 42.

The interface surface 42, to facilitate the connection between cartridge 41 and interface seat 40, is preferably a rigid rack.

Conversely, preferably, the bags 43 are deformable. Furthermore, in more detail, each bag 43 comprises walls made of a double barrier film permeable only to gases. In order to facilitate coupling of the bags 3 to the inlets 40a or plugs 42a, each bag 43 comprises a spout **43a.** The spout 43a is, therefore, configured to be housed in a respective plug 42a or a respective inlet 40a of the interface surface 42.

At the same time, each nozzle 43a can include a suction valve.

In particular, the bags 43 may include at least one maximum and one minimum calibrator comprising fluids having known physico-chemical characteristics to be analysed and respectively a high and low content of the same physico-chemical characteristics. Said high and low contents are preferably respectively close to the maximum and minimum contents with respect to the presumed values obtainable with the analysis.

More precisely, the maximum calibrator consists of a fluid having a pH and a sodium, potassium, ammonium, and chloride content known and preferably higher than the maximum value that can be found in a urine sample, while the minimum calibrator consists of a fluid having a pH and a sodium, potassium, ammonium, and chloride content known and preferably lower than the minimum value that can be found in a urine sample.

In addition, cartridge 41 can advantageously comprise a casing **44.**

If present, the casing 44 contains the bags 43.

Preferably, the casing 44 is made of recyclable material, e.g. cardboard. Therefore, casing 44, once emptied from bag 43, can be disposed of ecologically or used again.

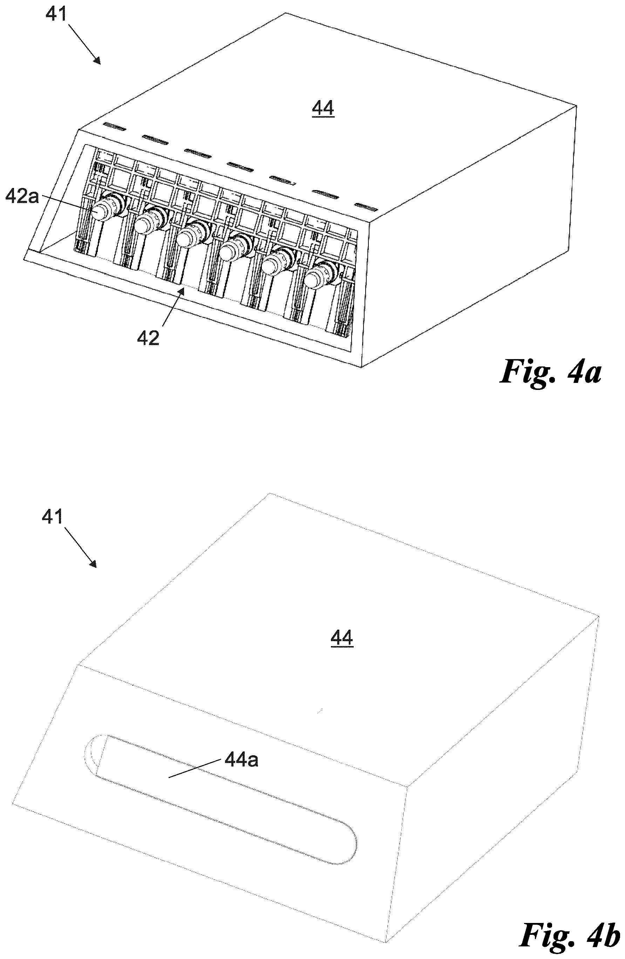

Furthermore, the casing 44 preferably defines an open container shape and therefore preferably includes at least one opening **44a.** The opening 44a faces, therefore, the interface surface 42. In particular, the opening 44a may be open over the entire interface surface 42 so that the interface surface 42 entirely faces the interface surface 42, as shown for example in Fig. 4a.

Alternatively, the opening 44a may face precisely the interface surface 42 at the plugs 42a, as shown for example in Fig. 4b. Furthermore, the opening 44a may be provided with a cover, e.g. a tear-off cover, suitable for covering the plugs 42a or the inlets 40a when the cartridge 41 is not in use and removable to allow the cartridge 41 to be connected to the interface housing 40.

At the same time, the interface housing 40 may comprise a flat bottom, on which the inlets 40a or plugs 42a are cut. Or, the interface housing 40 may comprise a guide **40b.** The guide 40a may be, for example, a bottom strip protruding from the rest of the bottom so as to make a raised part.

If present, the guide 40b is positioned at the inlets 40a, or plugs 42a, of the interface housing 40. In addition, the guide 40b is advantageously counter-bent at the opening 44a. In this way, the guide 40b allows the enclosure 44 to be clamped onto the guide 40b when the enclosure 40 is inserted into the interface housing 40. Since calibration or cleaning solutions are mainly liquid substances, the interface seat can advantageously include a **40c** drain hole.

The drain hole 40c, if present, is preferably a hole in the bottom of the interface seat 40 and preferably in the part not occupied by the guide 40b if the latter is present. Thus, the drain hole 40c is configured to allow liquid to flow out of the interface seat 40. This means that, should there be unwanted leakage from the interface surface 42, the drain hole 40c could allow excess liquid to flow out.

In general, the interface seat 40 is appropriately connected to the rest of the analysis apparatus 4 and the control means 5.

In order to enable the conveyance of urine between the various parts of the device 1, the control means 5 comprise not only electronic connection means for controlling the various valves, solenoid valves and other components of the device 1, but also include connectors for fluid passage between the various parts, for example between the first sampler module 2 and the analysis apparatus 4.

For example, overall, the control means 5 are placed in fluid passage connection with at least the first sampler module 2 and the analysis apparatus 4.

The means of control 5 may, therefore, include a steering station **50.**

If present, the steering station 50 is suitable for controlling device 1.

The steering station 50 may also include, or be connected to, an export element, such as a printer or mass storage device connection to allow results to be printed and/or viewed on an external device such as a computer.

In addition, steering station 50 can allow analyses to be programmed at pre-set times or, alternatively, analyses to be programmed at pre-set time intervals and, thus, the regular operation of device 1 and, therefore, the correct performance of the analysis without the presence of the operator.

The steering station 50 may, in this regard, comprise a screen or other similar element capable of allowing at least the results of the analysis to be displayed and control elements, such as a keyboard, capable of allowing the operation of the entire device 1 to be controlled. Alternatively, the screen may be of the touchscreen type and permit, in addition to the display of data, to control the device 1.

The control means 5 can be connected to the suction valves of the bags 43, when the interface surface 42 of the cartridge 41 is connected to the plugs 42a or the inlets 40a of the interface seat 40.

In addition, the control means 5 can be connected to the first pilot valve 26.

In this way, the control means 5 can control the flow in and out of the analysis apparatus 4.

Control means 5, in particular, may include a pump **51.**

The pump 51 can be operatively connected to at least the first pilot valve 26 and, therefore, can be placed in fluid passage connection with the first pilot valve 26.

In addition, the pump 51 can be connected to bag suction valves 43 when cartridge 41 is connected to interface seat 40.

Thus, the pump 51 may be configured to convey, in a controlled manner, urine from the first sampler module 2, i.e. from the containment part 20a to the analysis apparatus 4, or it may be configured to convey calibration or cleaning fluid from the bags 43, i.e. from the cartridge 41, to the rest of the analysis apparatus 4.

In a preferred, but not exclusive, form of realisation, the device 1 includes acquisition means **6.**

If present, the acquisition means 6 are operationally connected to the bag 3.

In addition, they are configured to acquire at least a first parameter related to the weight of the second urine portion, i.e. the urine portion exiting the first sampler module 2 via the release part 20b.

Thus, the control means 5 are preferably further operatively connected to the acquisition means 6. Furthermore, the control means 5 are advantageously configured to calculate a second parameter relating to the volume of the second urine portion in relation to the first parameter given a predetermined third parameter relating to the urine density.

Basically, the control means 5 allow, for example by means of the steering station 50, to set a known urine density value which constitutes the third parameter. Thus, knowing the weight of the second urine portion by means of the acquisition means 6, i.e. by acquisition of the first parameter, it is possible to calculate the volume of the second urine portion given simply by the ratio between the weight, i.e. the first parameter, and the density, i.e. the third parameter, multiplied by the gravitational acceleration.

Once the second parameter, i.e. the total volume of the second urine portion, has been obtained, it is possible to sum the second parameter with the predetermined volume of the containment part 20a, which contains the first urine portion.

In this way, control means 5 can obtain the total volume of urine in device 1.

In addition, the acquisition media 6 can be further configured to acquire a fourth parameter related to the urine flow time within the first drainage duct 20.

For example, it is possible to use a photocell that times how long urine passes through a section of drainage duct 20.

Thus, control means 5 can be further configured to relate the total volume of urine to the fourth parameter in order to determine a flow rate of urine.

The device 1 can also include a second sampler module **7.**

The second sampler module 7, if present, is operatively connected to the analysis apparatus 4. Thus, the second sampler module 7 may include a shut-off valve **70** in fluid passage connection with the analysis apparatus 4.

The shut-off valve 70 is preferably a reactive valve, designed to allow fluid flow connection when a specific device is connected to the shut-off valve 70 itself.

For example, shut-off valve 70 can be configured to allow the attachment of a Luer or Luer lock syringe.

In general, the shutoff valve is configured to accommodate a syringe attachment including sampled urine so that the analysis apparatus 4 can analyse the sampled urine.

Basically, therefore, the second sampler module 7 allows 1 urine that has already been sampled in a syringe to be introduced into the device.

The shut-off valve 70 is preferably operatively connected to the control means 5. In addition, the shut-off valve 70 may comprise second sensor means **70a.**

The second half-sensors 70a, if present, are advantageously optical. Furthermore, they are advantageously configured to detect the presence of a syringe engaged in the shut-off valve 70.

Thus, the second sampler module 7 may further comprise a second pilot valve **71.** The second pilot valve 71, like the first pilot valve 26, may be operatively connected to the pump 51. Thus, the control means 5 may be configured to actuate the second pilot valve 71 and the pump 51 whenever a syringe is engaged in the shut-off valve 70.

The second pilot valve 71, like the first pilot valve 26, can be a solenoid valve.

The pump 51, in detail, can be a peristaltic pump.

The sampler module 7 may also comprise a flushing valve **72.** If present, the flush valve 72 is preferably operatively connected to the pump 51 and allows the flushing of any urine residue remaining in the shut-off valve 70 and/or in the fluidic section connecting to the analysis apparatus 4. The flushing valve 72 may also be a solenoid valve.

By flushing, it avoids fouling due to salinity of the urine in the area of the shut-off valve 70 and prevents pollution between urine from different users.

From a structural point of view, the device 1 may include a support structure **10.** The support structure 10 may essentially be a frame or container or any other element that allows one or more of the components of the device 1 to be supported.

The support structure 10 is, therefore, configured at least to support the first sampler module 2, the bag 3, the analysis apparatus 4 and the control means 5.

In addition, the support structure 10 can also be configured to support, if present, the acquisition media 6 and the second sampler module 7.

In particular, advantageously, bag 3, in addition to being decoupled from the first sampler module 2, as explained above, is removably attached to the support structure 10.

In particular, the bag 3 is removably constrained to the structure 10 by means of constraining means **36.** The constraining means 36 are preferably resolvable. Thus, they may include at least one hook **36a** and one slot **36b.**

The hook 36a is preferably attached to the structure 10. The slot 36b is made on the bag 3 and configured to be threaded onto the hook 36a.

Naturally, the slot 36b could be made on the support structure 10 and the hook 36a could instead be attached to the bag 3.

In addition, any type of equivalent constraining means 36 may be used.

The described form of realisation is particularly advantageous when device 1 is equipped with acquisition means 6.

In fact, the latter may include a load cell **60.** The load cell 60, itself known, is preferably constrained on the support structure 10. Thus, the bag 3 may be removably constrained precisely to the load cell 60 via the detachable constraining means 36.

This means that, more specifically, for example, the hook 36a can be operatively connected to the load cell 60. Thus, the loop 36b made on the bag 3, being configured to be threaded onto the hook 36a, allows the bag 3 to be hung on the hook 36a in such a way as to transfer to the load cell 60 the weight of the contents of the bag 3 itself, for example of the second portion of urine when it reaches the bag 3.

In addition, to the bag 3, and irrespective of whether or not the bag 3 is connected to the support structure 10, the first sampler module 2 may also be removably constrained to the support structure 10. Furthermore, the first sampler module 2 can also be decoupled from the analysis apparatus 4.

In this regard, the structure 10 may include a housing **10a.**

The housing 10a can, therefore, be configured to contain the first sampler module 2.

First sampler module 2 and bag 3 may be independent of each other. Or, they may be mutually connected by means of connection means **32.**

The connection means 32 may be resolvable or unresolvable.

For example, the connection means 32 may comprise at least one strap **32a.** The strap 32a is advantageously flexible and is configured to allow the bag 3 and the first sampler module 2 to be supported on opposite sides of a hospital bed rail. Naturally, the connection means 32 may also comprise a plurality of straps 32a. The latter can be unfastened by means of buttons, or threaded into special hollow guides formed on the first sampler module 2 and/or the bag 3.

The support structure 10 may additionally include a compartment **10b.**

The compartment 10b may be arranged close to the shut-off valve 70. Even more in detail, the compartment 10b may include the shut-off valve 70, for example at a back wall. Thus, the compartment 10b is configured to accommodate, preferably stably, at least part of the tip of a said syringe.

Therefore, the compartment 10b can be counter-shaped to a syringe tip.

The support structure 10 may further comprise a plurality of conduits and/or fittings part of the control means 5 and capable of connecting in fluid passage connection at least part of the components of the device 1 as well as, naturally, the pump 51 or other similar organ capable of moving the fluid to be analysed.

The support structure 10 can also support steering station 50.

The device 1, and therefore, the control structure 10 may have a power supply system, not shown in the figures, capable of supplying power to the components of the device 1, such as the control means 5 and the analysis apparatus 4, and constituted by a battery and/or a connection cable capable of connecting the device to an external power supply network.

The operation of urine analysis device 1, described above in a structural sense, is as follows.

Initially, the device 1 is prepared by inserting cartridge 41 into interface seat 40.

In particular, the cartridge 41 causes plugs 42a to be coupled to inlets 40a.

Once this operation is completed, device 1 is ready to be used and the fluid sample, typically urine, to be analysed can be introduced via the first sampler module 2 or via the second sampler module 7.

In detail, in the case of the use of the second sampler module 7, the sampled urine is manually inserted by a medical or paramedical operator, e.g. by inserting a syringe into the compartment 10b engaged in the shut-off valve 70.

Alternatively, introduction takes place via the first sampler module 2, which, being connected directly to the patient via catheter, allows the urine to flow directly into the device 1.

In this case, the urine arrives via the cannula inlet 22 in the first tank 23, where it is conveyed to the first drainage duct 20. In particular, the urine first arrives in the containment part 20a, where a first portion of urine defined by the predetermined volume is collected, then the urine may flow out of the release part 20b if it exceeds the volume contained in the containment part 20a. In some cases, if the flow of fluid arriving in the first sampler module 2 is excessive and the level of the first collection tank 23 exceeds a predetermined level, the excess portion of fluid, i.e. the portion above said level, is directly expelled from the second drainage duct 24, acting as an overflow drain, directly into the bag 3 preventing said excess quantity from altering the proper functioning of the first sampler module 2.

The fluid in the first tank 23 may also pass through the first sensor means 25 under the action of gravity before reaching the first drainage duct 20. Urine passing through the first optical sensor means 25, for example, may interrupt a light beam, i.e. a continuous signal emitted by an optical emitter and detected by a photosensitive element.

In detail, the urine, interposing itself between the emitter and the photo-sensitive element interrupts the beam of light incident on the element and, therefore, the control means 5, suitably placed in current/data passage connection with the photosensitive element of the first sensor means 25, detect the interruption of the continuous signal and interrupt the reading of said continuous signal for a time at least equal to the interruption time. The latter time can be sampled by control means 5 to determine the fourth parameter.

Specifically, once through the first sensor media 25, the fluid to be analysed reaches the collection siphon, defined by the containment part 20a where a first portion of urine is collected and from which a second portion of urine exceeding the predetermined volume reaches the release part 25b and is then discharged into the bag 3.

In detail, the first portion of the fluid, corresponding to the quantity to be analysed, remains inside the containment part 20a thanks to the conformation thanks to the ventilation duct 27 which, by putting the ends of the containment part 20a and the external environment in communication with each other, allows the said ends to have the same pressure and therefore the fluid to remain between the two pressures.

At this point, the device 1 is ready to perform the analysis of the urine sample. Naturally, thanks to the acquisition media, the control means 5 are able to assess the total volume of urine that has passed through the device.

In fact, since the bag 3 is suspended from load cell 60 by hook 36a, load cell 60 is able to determine the weight of the second portion of urine collected in the bag.

Having obtained the weight, which defines the first parameter, it is possible, knowing a third parameter relating to the density of the urine and the gravitation acceleration which is a known constant, to determine the second parameter relating to the volume of the second portion of urine. Since the volume of the first portion of urine is known from the predetermined volume of the containment part 20a, by adding the second parameter to the predetermined volume it is possible to know the total volume of urine without having to adopt special sensor means or other elements such as drippers.

Furthermore, by relating the total volume to the flow time of the urine in the first sampler module 2 it is also possible to know the flow rate of the urine.

However, before first use or after a certain number of analyses have been performed, device 1 may require calibration.

To carry out the said calibration, initially the maximum and minimum calibrator are analysed, i.e. a complete analysis of a maximum and a minimum calibrator sample is carried out.

In particular, this analysis is carried out at a standard temperature set by thermostat 48 and, preferably, this temperature is around 37°C.

Therefore, through the control means 5, a fluid sample, e.g. of maximum calibrator, is taken from the relevant bag 43 of the cartridge 41 and sent to the analysis station of the analysis apparatus 4 and, more precisely, into the conduit formed by the containment chambers 47and the electrochemical sensors 47.

In particular, each ion-selective membrane 47b selects at least one respective ion and, in some cases, together with the same respective ion, similar ions.

Accordingly, each ion-selective electrode 47 reacts with the respective ion and possibly with said similar ions. Depending on the amount or concentration of the ions with which each ion-selective electrode 47 interacts, the same electrode 47 measures a potential difference with respect to the reference electrode 46, determining a control parameter.

The said control parameter is a function of the selected ion and the said similar ions, if any. The device 1 is therefore capable of interpreting said control parameters and extrapolating from them the physico-chemical characteristics of the analysed sample.

It has been determined that ion-selective electrodes 47 are capable of selecting and measuring the respective ions consisting of: H+ ions (for pH measurement), K+ ions (for Potassium measurement), CI- ions (for Chloride measurement), NH4+ ions (for Ammonium measurement). They select and measure the respective ions by themselves. The concentration of these ions is then directly proportional to the potential difference measured by the respective ion-selective electrode 47. From the control parameters obtained from the ion-selective electrodes 47 relating to H+, K+, CI- and NH4+, the concentration of the respective ions can thus be obtained directly. In contrast, the control parameter measured by the ion-selective electrode 47 relative to the Na+ ion (for the measurement of Sodium) is a function of the concentration of Na+ ions and also of H+ ions. In particular, the concentration of Sodium can be obtained from the respective control parameter and is dependent on a mathematical algorithm of the concentration of H+ ions (The concentration of H+ ions can in turn be obtained directly from the respective ion-selective electrode 47, as mentioned above).

Furthermore, the control parameter measured by the ion-selective electrode 47 relative to the NH4+ ion (for ammonium measurement) is a function of the concentration of NH4+ ions and also of K+ ions. In particular, the ammonium concentration is obtainable from said relative control parameter minus a mathematical algorithm of the K+ ion concentration (The K+ ion concentration is in turn obtainable directly from the respective ion-selective electrode 47, as mentioned above).

To summarise, the control means 5 first determine said control parameters, at least one of which directly determines at least one of the values of the physico-chemical characteristics to be determined. Subsequently, the same means of control 5 determine further physico-chemical characteristics depending on said first parameters and the physico-chemical characteristics already determined. Consequently, the control means 5, based on the signals sent to electrodes 46 and 47, quantifies this potential difference and thus determines the results of the analysis, i.e., in this example, the pH and the contents of sodium, potassium, ammonium, and chloride in the urine sample.

In conclusion, at the end of the analysis of the sample of maximum, the control means 5 determine, for each of the physico-chemical characteristics, a maximum pair in which the content of a physico-chemical characteristic and the control parameter relating to the same characteristic obtained by analysing the maximum calibrator are indicated. For example, the maximum torque relating to pH identifies two distinct values, namely the pH content and the potential difference determined by the relevant ion-selective electrode 47.

Once the analysis of the maximum calibrator sample has been completed and the aforementioned maximum torques have been obtained, the control means 5 command the ejection of the maximum calibrator sample, which is sent to one of the bags 43 in the cartridge 41 to collect the rejects.

Once unloading is complete, the second calibration is performed, which, using the minimum calibrator, makes it possible to determine, for each physico-chemical characteristic, a minimum torque formed by the relative control parameter and the relative value obtained with the minimum calibrator.

Having completed the above two calibration analyses, the control means 5 can carry out a linearisation of the values between the maximum and minimum torque for each physico-chemical characteristic. Linearisation means that the control means 5 assigns a linear course between the maximum and minimum torque, i.e. they assign a constant course/variation of the value of the physico-chemical characteristic, preferably on a logarithmic basis, with respect to the control parameter.

In particular, the control means 5 can perform for each physico-chemical characteristic a linearisation on a logarithmic scale, i.e. a linearisation between the first parameter and the logarithm of the value of the physico-chemical characteristic. Once the linearisation is complete, the device 1 is calibrated and ready to do the analysis, and, for example via steering station 50, the operator can select the physico-chemical characteristics he or she wishes to analyse and start the analysis operation.

The control means 5 controls the entry of the urine to be analysed into the conduit formed by the containment chambers 47e and performs the analysis of the urine sample at a temperature of approximately 37°C, appropriately controlled by the thermostat 48.

Initially, the control parameter is determined for each physico-chemical characteristic, i.e. the potential difference between the reference electrode 46 and the ion-selective electrode 47.

Subsequently, the control means 5 calculate the individual physico-chemical characteristics by comparing the control parameters obtained from the analysis of the above-mentioned sample with the above-mentioned linearisation.

In particular, for each physico-chemical characteristic, control medium 5 reports a control parameter relating to a characteristic on the relevant linearisation and, based on this, derives the corresponding value of the physico-chemical characteristic in question.

For example, in the case of calculating pH, the electrochemical sensor corresponding to pH detects the potential difference between electrodes 46 and 47, i.e. the pH control parameter. This control parameter is then processed by control medium 5, which plots the parameter on a graph obtained in the linearisation, derives the pH torque and thus obtains the pH value.

Once the analysis is complete, the results are displayed on the screen, printed and/or stored on special mass storage devices.

The control means 5 control the ejection from the analysis station of the fluid to be analysed, i.e. urine, which is collected in the cartridge 41 in a bag 43.

Once the bags 43 are exhausted, i.e. when the bags 43, containing calibrators or the flushing fluid, have exhausted their contents and/or the bags 43, designed to collect the waste fluid, are full, cartridge 41 is removed and replaced.

A similar sequence is repeated with the control solutions in the bags 43 of the cartridge 41, which have the function of verifying the instrumental quality status over time by means of the analysis apparatus 4 and the control means 5, as mentioned above.

The invention also comprises a new process for urine analysis. The process is advantageously implemented by the above-described device 1.

In brief, the process comprises an initial acquisition phase.

At this stage, an initial parameter relating to the weight of the second portion of urine in bag 3 is acquired by means of acquisition means 6.

In addition, the procedure includes a calculation step, in which a second parameter relating to the volume of the second portion of urine in the bag 3 is calculated by means of the control means 5. The second parameter, as already mentioned, is calculated in relation to the first parameter given a third predetermined parameter relating to the density of the urine and, naturally, known gravitational acceleration.

In addition, the procedure includes an addition step. In the addition step, the second parameter is added with the predetermined volume of the containment part 20a to obtain the total urine volume.

The procedure may further comprise, a second acquisition step in which a fourth parameter relating to the urine flow time within the first drainage duct 20 is acquired by means of the acquisition means 6. Thus, the procedure may comprise a splitting step. In the splitting step, the total volume of urine is related, by means of the control means 5, to the fourth parameter so as to determine a urine flow rate.

Naturally, the process may include further steps.

For example, initially, the procedure may involve a calibration phase in which, using the maximum and minimum calibrators as described above, device 1 is calibrated for analysis.

This stage involves an initial analysis step in which the control parameters of each physical characteristic are determined. In detail, at this stage, the potential differences between electrodes 46 and 48 are preferably determined for each characteristic.

The calibration phase thus comprises a maximum calibration step and a minimum calibration step in which a maximum torque and a minimum torque are obtained for each characteristic under consideration. In detail, each torque identifies the value of that characteristic as a function of a control parameter which, in the case of electrochemical analysis, is the potential difference between the reference electrode 46 and the ion-selective electrode 47.

These maximum and minimum pairs are composed of the control parameter obtained in the above-described step and the maximum value, e.g. obtained by interpolation. In particular, the pairs are calculated taking into account not only the control parameter corresponding to the characteristic in question, but also control parameters and/or values relating to at least one other characteristic that could influence and thus alter the results of the analysis.

In these stages, maximum and minimum pairs of pH and sodium, potassium, ammonium and chloride content can therefore be determined by interpolating the various control parameters. In detail, the value of the sodium content is determined on the basis of the control parameter relating to sodium content and the first parameter relating to pH, while the value of the ammonium content is determined on the basis of the control parameter relating to ammonium content and the control parameter relating to potassium content.

After the above two calibration steps, this phase is concluded by the linearisation step in which the values between maximum and minimum torque are linearised, as described above.

Thereafter, there is an inlet phase in which, for example through the first sampler module 2 a quantity of urine is fed into the device. Alternatively, this feed phase can be carried out either in parallel with or prior to the calibration phase.

Once the sample has been inserted and device 1 has been calibrated, the analysis phase comprising two sub-phases is started.

In the first analysis sub-stage, the electrochemical analysis of the sample is performed and the control parameter, i.e. the potential differences between the reference electrode 46 and the ion-selective electrode 47, is determined for each physico-chemical characteristic to be analysed.

Once these potentials have been identified, the first analysis sub-step is concluded and the second analysis sub-step begins, in which the value of each characteristic is determined by comparing the control parameters obtained in the first analysis sub-step with the result of the aforementioned linearisation step.

Finally, the procedure may provide for a plurality of the above-mentioned analysis phases carried out at fixed time intervals so as to allow for the patient's status to be monitored on a substantially continuous basis.

The urine analysis device 1 according to the invention achieves important advantages.

In fact, the analysis device makes it possible to assess all physico-chemical parameters of interest related to the urine sample, always knowing the total volume of urine assessed. In particular, the volume determination is performed in a simple, automatic and autonomous manner.

Therefore, it is not necessary to know the initial amount of urine sampled.

In addition, it is also possible to use urine that has already been sampled, e.g. by syringe, thanks to the second sampler module 7.

The latter allows stable and efficient coupling of a syringe, whatever the coupling, thanks to the shut-off valve 7 and compartment 10b.

In addition, the shut-off valve 70 at the end of the analysis is flushed by operating the flushing valve 72 and by pump 51 sucking cleaning solution from a bag 43 of the cartridge 41. This allows urine from different patients to be analysed without contaminating each other and also avoids any fouling in the area of the shut-off valve 70 due to the salt content of the urine.

In addition, the first sampler module 2 and bag 3 can be removed from support structure 10, thus allowing at least part of the same device 1 to be used for another patient by simply replacing first sampler module 2 and bag 3.

In addition, the fact that bags 43 are collected in a single cartridge 41 makes management of the calibration and cleaning block simple and achievable by any user, even a non-specialist.

In general, therefore, the device 1 allows simple, fast and frequent analyses.

In addition, the device 1 does not require the presence of medical and paramedical personnel and, therefore, enables analyses to be carried out essentially automatically.

The invention is susceptible to variations within the inventive concept as defined by the claims. Here, all details can be replaced by equivalent elements and the materials, shapes and sizes can be any.

## Claims

1. Urine analysis device (1) capable of analysing a urine sample to determine the value of physico-chemical characteristics of said urine sample and comprising:
- a first sampler module (2) configured to be placed in fluid passage connection with a user and including a first drainage duct (20) comprising
- a containment part (20a) defining a predetermined volume and configured to retain an initial portion of said urine, and
- a release part (20b) configured to convey a second portion of said urine out of said first sampler module (2);
- a diuresis bag (3) in fluid passage connection with at least said release part (20b) and configured to collect said second portion of urine;
- an analysis apparatus (4) placed in fluid passage connection with said containment part (20a) and configured to analyse at least part of said first portion of urine;
- control means (5) operationally connected to said analysis apparatus (4) and configured at least to process data collected by said analysis apparatus (4); and **characterised by** further comprising
- a second sampler module (7) operatively connected to said analysis apparatus (4) and including a shut-off valve (70) in fluid passage connection with said analysis apparatus (4) and configured to accept a syringe entry including sampled urine so that said analysis apparatus (4) can analyse said sampled urine.

2. Device (1) according to claim 1, wherein said shut-off valve (70) is configured to allow engagement of a Luer or Luer lock type syringe.

3. Device (1) according to any one of the preceding claims, wherein said shut-off valve (70) is operatively connected to said control means (5) and comprises second optical sensor means (70a) configured to detect the presence of a said syringe engaged in said shut-off valve (70).

4. Device (1) according to any one of the preceding claims, wherein said second sampler module (7) further comprises a second pilot valve (71) and said control means (5) comprise a pump (51) operatively connected to at least said pilot valve (71) and wherein said control means (5) are configured to actuate said second pilot valve (71) and said pump (51) whenever a said syringe is engaged in said shut-off valve (70).

5. Device (1) according to the preceding claim, wherein said second sampler module (7) further comprises a flushing valve (72) operatively connected to said pump (51) and wherein said control means (5) are configured to actuate said second flushing valve (72) and said pump (51) whenever said pilot valve (71) has been actuated.

6. Device (1) according to any one of claims 4-5, wherein said second pilot valve (71) and/or said flushing valve (72) each include a solenoid valve and said pump (51) is a peristaltic pump.

7. Device (1) according to any one of the preceding claims, comprising a support structure (10) configured to support said first sampler module (2), said bag (3), said analysis apparatus (4), said control means (5) and said second sampler module (7) and wherein said support structure (10) comprises a compartment (10b) configured to accommodate at least part of the tip of a said syringe and including said shut-off valve (70).

8. Device (1) according to any preceding claim, wherein said first sampler module (2) comprises an cannula inlet (22) configured to be connected to said user, a first tank (23) in fluid passage connection with said cannula inlet (22) and said containment part (20a) and a second drainage duct (24) in fluid passage connection with said first tank (23) and said cannula outlet (21) and configured to convey said urine from said first tank (23) to said cannula outlet (21) when said urine in said first tank (23) exceeds a predetermined threshold level.

9. Device (1) according to claim 7, wherein one between said cannula inlet (22) and said first drainage duct (20) comprises first optical sensor means (25) operatively connected to said control means (5) and configured to detect the passage of said urine through said cannula inlet (22) or said first drainage duct (20), wherein said first sampler module (2) further comprises a first pilot valve (26) operatively connected to said control means (5) and wherein said control means (5) are configured to actuate said first pilot valve (26) when said urine passes through said cannula inlet (22) or said first drainage duct (20).

10. Device (1) according to any preceding claim, further comprising acquisition means (6) operatively connected to said bag (3) and configured to acquire at least a first parameter relative to the weight of said second urine portion, and wherein said control means (5) are further operatively connected to said acquisition means (6) and configured to calculate a second parameter relating to the volume of said second urine portion in relation to said first parameter given a third predetermined parameter relating to the density of said urine, and to sum said second parameter with said predetermined volume of said containment part (20a) to obtain the total volume of said urine.

11. Device (1) according to any preceding claim, wherein said support structure (10) is configured to also support said acquisition means (6) and said acquisition means (6) comprise a load cell (60) constrained on said support structure (10) and said hook (36a) operatively connected to said load cell (60) comprise a load cell (60) constrained on said support structure (10) and said bag (3) is removably constrained to said load cell (60) by detachable constraining means (36).
